# EUROPÄISCHE PATENTSCHRIFT

(11) **EP 3 516 388 B1**
(45) Veröffentlichungstag und Bekanntmachung des Hinweises auf die Patenterteilung: **12.06.2024**
(21) Anmeldenummer: 17765194.0
(22) Anmeldetag: 18.09.2017
(51) Int. Cl.: G01N 21/80, G01N 33/18, G01N 31/22

(54) **VERFAHREN ZUR BESTIMMUNG VON AMMONIUM**
METHOD FOR DETERMINING AMMONIUM
PROCÉDÉ DE DOSAGE D'AMMONIUM

(30) Priorität: 20.09.2016 EP 16189652
(43) Veröffentlichungstag der Anmeldung: 31.07.2019
(73) Patentinhaber: Merck Patent GmbH, 64293 Darmstadt (DE)
(72) Erfinder: OLT, Ralf, 64750 Luetzelbach-Seckmauern (DE)
(74) Vertreter: Merck Patent Association
(86) Internationale Anmeldenummer: PCT/EP2017/073384
(87) Internationale Veröffentlichungsnummer: WO 2018/054797

(56) Entgegenhaltungen:
- PATTON C J ET AL: "SPECTROPHOTOMETRIC AND KINETICS INVESTIGATION OF THE BERTHELOT REACTION FOR THE DETERMINATION OF AMMONIA", ANALYTICAL CHEMISTRY, AMERICAN CHEMICAL SOCIETY, US, Bd. 49, Nr. 3, 1. März 1977 (1977-03-01), Seiten 464-469, XP000670548, ISSN: 0003-2700, DOI: 10.1021/AC50011A034
- Phillip L Searle: "The berthelot or indophenol reaction and its use in the analytical chemistry of nitrogen. A review", Analyst, 1. Januar 1984 (1984-01-01), Seiten 549-568, XP055429658, DOI: 10.1039/AN9840900549 Gefunden im Internet: URL:http://pubs.rsc.org/en/content/article pdf/1984/AN/AN9840900549 [gefunden am 2017-11-28] in der Anmeldung erwähnt

## Beschreibung

Die vorliegende Erfindung betrifft ein Verfahren zur Bestimmung von Ammonium bzw. Ammoniak in wässrigen Proben nach der bekannten Berthelot Methode, wobei die Gefahr von falsch niedrigen Ergebnissen durch die zusätzliche Messung der Extinktion im Absorptionsbereich des als Katalysator eingesetzten Nitroprussids stark reduziert wird.

### Stand der Technik

Ammonium-Stickstoff (NH₄-N) ist in vielen oberirdischen Gewässern, in einigen Grundwässern, sowie in häuslichen und vielen gewerblichen Abwässern enthalten (Quelle: DIN 38406-5, ISO 7150-1).

Ammoniak ist für Fische schon in geringen Konzentrationen giftig. Ammoniumgehalte im Wasser von 0,5 bis 1 mg/l werden deshalb, je nach pH-Wert des Wassers, als bedenklich für Fische eingestuft. Bei Ammoniumgehalten von über 1 mg/l ist ein Gewässer für Fischereizwecke typischerweise nicht geeignet.

Ammoniumverbindungen gehören demnach zu den wassergefährdenden Stoffen, Grenzwerte sind in vielen Regularien zu finden. Eine Kontrolle des Ammoniumgehaltes in Wasser- und Abwasserproben ist deshalb zwangsläufig regelmäßig erforderlich.

Häufig werden genormte Verfahren zur Ermittlung des Ammoniumgehaltes verwendet. Dazu gehört unter anderem das Berthelot-Verfahren.

Das Verfahren findet bei alkalischem pH-Wert statt. Dort liegt Ammonium als Ammoniak (NH₃) vor.

Im ersten Schritt des Berthelot Verfahrens reagiert Ammoniak mit Hypochlorit zu Monochloramin.

Im nächsten Schritt reagiert das entstandene Monochloramin in Anwesenheit des Katalysators Nitroprussid mit einem Phenolderivat zu einem Chlorchinon-monoimin. Als Phenolderivat kommen im Prinzip unterschiedliche Verbindungen in Frage. Häufig findet man in den offiziellen Verfahren z.B. Salycilate, Thymol oder 2-Chlorphenol.

Im letzten Reaktionsschritt reagiert das entstandene Chlorchinon-monoimin mit einem weiteren Molekül des Phenolderivates zu einem entsprechenden Indophenol.

Die blaue Farbe des entstandenen Indophenols wird photometrisch gemessen (bei einer Wellenlänge im Bereich des Absorptionsmaximums) und korreliert in bestimmten Messbereichen mit dem Ammoniumgehalt der Probe.

Der komplette Reaktionsmechanismus ist bis heute nicht eindeutig geklärt und läuft über viele Zwischenprodukte ab.

Weitere Informationen und Details zum Berthelot-Verfahren finden sich beispielsweise in Philip L. Searle, Analyst, May 1984, Vol 109, Seiten 549-568 "The Berthelot or Indophenol Reaction and Its Use in the Analytical Chemistry of Nitrogen" oder Michael D. Krom, Analyst, April 1980, Vol. 105 No. 1249, Seiten 305-316 "Spectrophotometric Determination of Ammonia: A Study of a Modified Berthelot Reaction Using Salicylate and Dichloroisocyanurate". Auch in Patton C.J. and Crouch S.R., "Spectrophotometric and Kinetics Investigation of the Berthelot Reaction for the Determination of Ammonia", Analytical Chemistry, Vol 49, No. 3, 1977, 464-469, finden sich Angaben zur Art und Durchführung des Berthelot-Verfahrens.

Das Verfahren kann im Prinzip für verschiedene Messbereiche verwendet werden, wenn man die Probe/Reagenzverhältnisse entsprechend anpasst oder die Probe vorverdünnt.

Ammonium Tests, die nach diesem Verfahren arbeiten, werden auch von verschiedenen Herstellern in Form von gebrauchsfertigen Testsätzen angeboten.

Das oben beschriebene Verfahren weist den Nachteil auf, dass die entstehende blaue Farbe nur in bestimmten Konzentrationsbereichen des zu bestimmenden Ammoniums mit dem Ammoniumgehalt korreliert.

In der Regel steigt das Messsignal (Extinktion) der photometrischen Messung mit zunehmenden Ammoniumgehalt der Probe an.

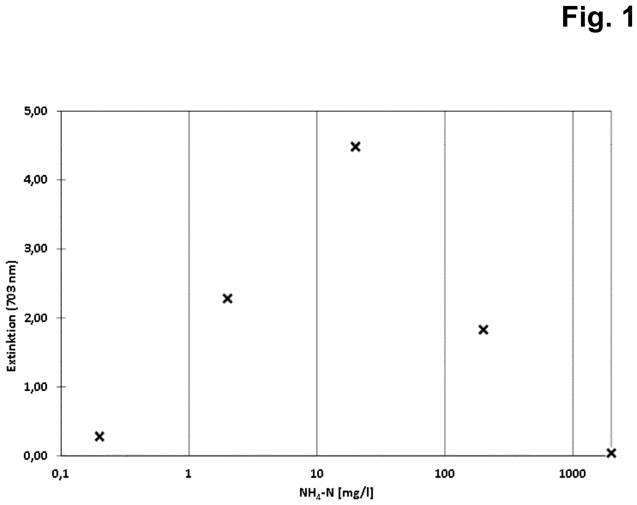

Es hat sich in der Praxis herausgestellt, dass bei sehr hohen Ammoniumkonzentrationen der Probe, das Messsignal, d.h. die Extinktion, wieder zurückgeht (siehe Abb. 1). Die Ursache ist bisher nicht eindeutig identifiziert. Vermutlich läuft der Reaktionsmechanismus bei ungünstigen Analyt/Reagenzverhältnissen nicht mehr komplett ab. Auch eine pH-Verschiebung durch zu viel entstehendes Monochloramin kann dafür verantwortlich sein. Eine zuverlässige Aussage über den Ammoniumgehalt ist in diesem Falle nicht möglich und es kann im schlimmsten Fall zu einer deutlichen Fehleinschätzung des Ammoniumgehaltes mit gravierenden Auswirkungen auf die Umwelt kommen.

Die Normen und Testsatzhersteller schreiben daher vor, dass zusätzlich zur Analyse der Probe weitere Analysen mit verdünnter Probe durchzuführen sind (verschiedene Verdünnungsstufen), um die Plausibilität des Messergebnisses zu überprüfen. Das ist für den Anwender unbequem und zeitaufwändig.

### Aufgabe der Erfindung

Aufgabe der vorliegenden Erfindung ist es daher, ein Verfahren zur Bestimmung des Ammoniumgehaltes von wässrigen Proben zur Verfügung zu stellen, das neben dem Messergebnis einer einzelnen Messung direkt auch Informationen zur Plausibilität dieses Messergebnisses liefert, ohne dass weitere Proben, z.B. im Rahmen einer Verdünnungsreihe, vermessen werden müssen.

### Lösung der Aufgabe

Es wurde gefunden, dass die Plausibilität einer Messung des Ammoniumgehaltes nach Berthelot überprüft werden kann, wenn neben der Messung der Extinktion im Absorptionsbereich des gebildeten blauen Indolfarbstoffes bei der Probe zusätzlich eine Messung der Extinktion im Absorptionsbereich des als Katalysator eingesetzten Nitroprussids vorgenommen wird. Zeigt die Messung im Absorptionsbereich des als Katalysator eingesetzten Nitroprussids an, dass ausreichend Nitroprussid vorhanden ist, ist das Ergebnis der Ammonium-Bestimmung plausibel. Zeigt die Messung im Absorptionsbereich des Nitroprussids an, dass wenig oder kein Katalysator mehr vorhanden ist, muss die Plausibilität des Ergebnisses der Ammonium-Bestimmung angezweifelt werden.

Auf diese Weise kann mittels einer direkt an derselben Probe durchgeführten zusätzlichen Messung der Extinktion im Absorptionsbereich des Nitroprussids die Plausibilität des eigentlichen Messergebnisses der Ammonium-Bestimmung überprüft werden. Weitere Messungen von Verdünnungen der Probe sind normalerweise nicht notwendig.

Gegenstand der vorliegenden Erfindung ist daher ein Verfahren zur Bestimmung des Ammonium-Gehaltes von wässrigen Proben, das neben dem Messergebnis einer einzelnen Messung direkt auch Informationen zur Plausibilität dieses Messergebnisses liefert, wobei
a) die Probe bei alkalischen pH-Wert mit einem Chlorierungsmittel, einem Phenolderivat und Nitroprussid versetzt wird,
b) die Extinktion der in Schritt a) erhaltenen Mischung im Absorptionsbereich des entstehenden blauen Farbstoffs und im Absorptionsbereich des Nitroprussids bestimmt wird, wobei aus der Extinktion im Absorptionsbereich des entstehenden blauen Farbstoffs der Ammonium-Gehalt bestimmt wird und aus der Extinktion im Absorptionsbereichs des Nitroprussids die Plausibilität ermittelt wird.

In einer bevorzugten Ausführungsform ist das Chlorierungsmittel Dichlorisocyanursäure.

In einer bevorzugten Ausführungsform ist das Phenolderivat 2-Chlorphenol oder Thymol, besonders bevorzugt 2-Chlorphenol.

In einer bevorzugten Ausführungsform wird der alkalische pH in Schritt a) erzeugt durch Zugabe von Natronlauge.

In einer bevorzugten Ausführungsform liegt der pH-Wert in Schritt a) zwischen pH 11 und pH 12. Der optimale pH-Wert für eine Probe ist abhängig vom verwendeten Phenolderivat, bei 2-Chlorphenol ist beispielsweise ein pH-Wert zwischen pH 11,5 und 11,8 bevorzugt.

In einer bevorzugten Ausführungsform erfolgt die Messung der Extinktion in Schritt b) mit einem Spektralphotometer.

In einer bevorzugten Ausführungsform erfolgen die Bestimmungen der Extinktionen derart, dass eine Messung in dem Wellenlängenbereich zwischen 350 und 450 nm vorgenommen wird, und eine Messung in dem Wellenlängenbereich zwischen 600 und 800 nm.

Gegenstand der vorliegenden Erfindung ist weiterhin ein Verfahren zur Bestimmung der Plausibilität des Messergebnisses einer Bestimmung des Ammonium-Gehaltes von wässrigen Proben, wobei die Messung durchgeführt wird entsprechend dem erfindungsgemäßen Verfahren zur Bestimmung des Ammonium-Gehaltes von wässrigen Proben, d.h. durch
a) Erzeugen einer wässrigen Probe mit alkalischem pH-Wert und versetzen dieser Probe mit einem Chlorierungsmittel, einem Phenolderivat und Nitroprussid,
b) Bestimmung der Extinktion der in Schritt a) erzeugten Mischung, optional nach Abwarten einer Reaktionszeit, im Absorptionsbereich des entstehenden blauen Farbstoffs und im Absorptionsbereich des Nitroprussids,
und wobei die Extinktion der Probe im Absorptionsbereich des entstehenden blauen Farbstoffs nur dann als plausibles Messergebnis angesehen wird, wenn die Extinktion im Absorptionsbereich des Nitroprussids oberhalb eines vorab zu bestimmenden Schwellenwerts liegt, wobei der Schwellenwert der Extinktion, die die Hälfte des in der Mischung aus Schritt a) vorhandenen Nitroprussids in dieser Mischung hervorrufen würde, wenn diese Mischung kein Ammonium enthielte, entspricht.

In einer bevorzugten Ausführungsform wird der Schwellenwert bestimmt durch
i. Bereitstellen einer Blindwert-Probe, die kein Ammonium enthält, mit alkalischen pH-Wert und Versetzen dieser Blindwert-Probe mit einem Chlorierungsmittel, einem Phenolderivat und Nitroprussid, so dass sie bezüglich pH-Wert, Chlorierungsmittel, Phenolderivat und Nitroprussid dieselbe Zusammensetzung und Konzentration aufweist wie die Mischung, deren Extinktion in Schritt b) bestimmt wird
ii. Bestimmung der Extinktion der Blindwert-Probe im Absorptionsbereich des Nitroprussids, wobei die Extinktion des Nitroprussids in Schritt b) und Schritt ii bevorzugt bei derselben Wellenlänge bestimmt werden
iii. Berechnung des Schwellenwertes durch Halbieren des in Schritt ii) bestimmten Extinktionswerts.

In einer bevorzugten Ausführungsform ist das Chlorierungsmittel Dichlorisocyanursäure.

In einer bevorzugten Ausführungsform ist das Phenolderivat 2-Chlorphenol oder Thymol, besonders bevorzugt 2-Chlorphenol.

In einer bevorzugten Ausführungsform wird der alkalische pH in Schritt a) erzeugt durch Zugabe von Natronlauge.

In einer bevorzugten Ausführungsform liegt der pH-Wert in Schritt a) zwischen pH 11 und pH 12. Der optimale pH-Wert für eine Probe ist abhängig vom verwendeten Phenolderivat, bei 2-Chlorphenol ist beispielsweise ein pH-Wert zwischen pH 11,5 und 11,8 bevorzugt.

In einer bevorzugten Ausführungsform erfolgt die Bestimmung der Extinktion des Nitroprussids in Schritt b) und Schritt ii beim Absorptionsmaximum des Nitroprussids.

Die oben beschriebenen einzelnen Aspekte bzw. Gegenstände der Erfindung können auch in jeder beliebigen Kombination von zwei oder mehreren Aspekten bzw. Gegenständen verwirklicht werden.

Abbildung 1 zeigt die gemessene Extinktion der bei der Bestimmung des Ammoniumgehaltes einer Probe nach Berthelot für verschiedene Ammonium-Konzentrationen.

Abbildung 2 zeigt die gemessene Extinktion bei der Bestimmung des Ammoniumgehaltes einer Probe nach Berthelot für verschiedene Ammonium-Konzentrationen im Absorptionsbereich des entstehenden blauen Farbstoffs und im Absorptionsbereich des Nitroprussids.

Erfindungsgemäß ist eine wässrige Probe eine Probe, die ein oder typischerweise mehrere in Wasser gelöste Komponenten enthält. In der Regel enthält die wässrige Probe keine weiteren Lösungsmittel außer Wasser. Sie kann aber bis zu 20 % eines oder mehrerer mit Wasser mischbarer Lösungsmittel wie z.B. Ethanol enthalten. Eine wässrige Probe kann eine Wasser-Probe sein, ein Lebensmittel oder Getränk, oder eine Körperflüssigkeit. Sehr bevorzugt ist die wässrige Probe eine Wasser-Probe, wie z.B. eine aus einem Gewässer entnommene Probe, eine Abwasser-Probe, eine Grundwasser-Probe, eine Leitungswasser-Probe, eine Probe von Wasser, das einem industriellen Prozess zugeführt oder von einem industriellen Prozess als Abwasser abgeführt wird, etc.

Der Ammonium-Gehalt einer Probe ist der in einer Probe vorhandene Gehalt an Ammonium-Verbindungen, die beim alkalisch machen der Probe in Ammoniak übergeführt werden können bzw. in einer alkalischen Probe als Ammoniak vorliegen. Typischerweise wird die Probe dabei auf einen pH-Wert zwischen pH 10 und pH 13,5, bevorzugt zwischen 11 und 12 gebracht. Dies kann mit Basen oder Laugen erfolgen, beispielsweise mit wässriger LiOH-, NaOH-, KOH- Lauge, bevorzugt mit Natronlauge.

Beispiele für Ammonium-Verbindungen sind daher Ammonium-Salze, Ammoniumhydroxid und Ammoniak.

Bei der Bestimmung des Ammonium-Gehaltes nach Berthelot wird die Probe im Alkalischen mit einem Chlorierungsmittel, einem Phenolderivat und Nitroprussid als Katalysator versetzt und typischerweise eine bestimmte Zeit, die in der Regel zwischen 5 und 30 Minuten, bevorzugt zwischen 10 und 20 Minuten liegt, abgewartet, damit die Nachweisreaktion stattfinden kann. Dabei entsteht über mehrere Reaktionsschritte ein blau gefärbtes Indophenol. Die im Absorptionsbereich des Indophenols bestimmte Extinktion korreliert in bestimmten, dem Fachmann bekannten Messbereichen mit dem Ammoniumgehalt der Probe. Für die erfindungsgemäße Durchführung der Messung des Ammonium-Gehalts kann jede Variante der Berthelot-Methode verwendet werden, bei der Nitroprussid als Katalysator verwendet wird.

Geeignete Chlorierungsmittel sind beispielsweise Natriumhypochlorit und Dichlorisocyanurat (DIC) oder Dichlorisocyanursäure. Besonders bevorzugt ist Dichlorisocyanursäure.

Geeignete Phenolderivate sind beispielsweise Phenol oder an der 2-Position substituierte Phenole wie 2-Chlorphenol, Thymol oder Salicylate.

Beispielsweise kann das in DIN 38406/5 beschriebene Verfahren verwendet werden, bei dem statt des oft verwendeten Hypochlorits Dichlorisocyanursäure und statt Phenol Natriumsalicylat verwendet wird.

Nitroprussid, auch Natrium Nitroprussid genannt, ist ein Komplex der Summenformel Na₂[Fe(CN)₅NO]·2 H₂O.

Um den Ammoniumgehalt quantitativ zu bestimmen, wird die Extinktion der Probe nach Zugabe der notwendigen Reagenzien und nach Bildung des blau bis blau-grün gefärbten Indophenols bei einer bestimmten Wellenlänge im Absorptionsbereich des Indophenols bestimmt. Typischerweise erfolgt die Messung beim Absorptionsmaximum. Die quantitative Bestimmung kann dann mithilfe einer Eichkurve durchgeführt werden. Dieses Vorgehen ist dem Fachmann bekannt. Die Eichkurve und die Wellenlänge, bei der die Extinktion bestimmt werden, müssen für jede Reagenzienkombination (Art und Menge von Chlorierungsmittel, Phenolderivat, pH, etc.) entsprechend bestimmt werden. Typischerweise entstehen bei dem Nachweis Indophenole, die ein Absorptionsmaximum im Wellenlängenbereich zwischen 600 und 800 nm aufweisen.

Kern der vorliegenden Erfindung ist, dass die Plausibilität oder auch Richtigkeit des Ergebnisses einer Messung des Ammonium-Gehaltes einer wässrigen Probe nach dem Berthelot-Verfahren nicht wie bisher durch aufwändige Messung von Verdünnungsreihen dieser Probe bestimmt werden muss, sondern durch die Vermessung nur dieser einen Probe selbst bestimmt werden kann.

Dazu erfolgt die Bestimmung des Ammoniumgehalts nach Berthelot entsprechend bekannter Verfahren und mit den dem Fachmann bekannten üblichen Reagenzien, wobei als Katalysator Nitroprussid verwendet wird. Zusätzlich zu der üblichen Messung der Extinktion der Probe im Absorptionsbereich, bevorzugt im Absorptionsmaximum, des entstehenden Indophenols wird eine zweite Messung an derselben Probe im Absorptionsbereich von Nitroprussid, bevorzugt bei ca. 400 nm, durchgeführt.

Das Ergebnis der zweiten Messung im Absorptionsbereich des Nitroprussids erlaubt eine Aussage über die in der Messprobe enthaltene Menge an Nitroprussid. Es wurde gefunden, dass bei sehr hohen Gehalten an Ammonium, die zu einem falsch niedrigen Extinktionswert im Absorptionsbereich des Indophenols führen, zugleich auch die Menge an Nitroprussid und damit die Extinktion im Absorptionsbereich des Nitroprussids sehr gering ist. Durch diese Korrelation erlaubt die Bestimmung des Nitroprussid-Gehaltes in der Probe einen direkten Rückschluss auf die Plausibilität des Ergebnisses der Bestimmung des Ammoniumgehalts.

Da dem Fachmann bekannt ist, wieviel Nitroprussid zu der Probe zugegeben wurde, kann er aus dem Ergebnis der Messung im Absorptionsbereich des Nitroprussids entsprechende Rückschlüsse ziehen. Typischerweise kann davon ausgegangen werden, dass das Ergebnis der Ammoniumbestimmung plausibel ist, wenn die Messung im Absorptionsbereich des Nitroprussids ergibt, dass zumindest die Hälfte des ursprünglich der Probe zugegebenen Nitroprussids noch als solches vorhanden ist und damit zur Absorption in diesem Bereich beiträgt.

Bevorzugt erfolgt die Bestimmung der Plausibilität des erhaltenen Messergebnisses der Bestimmung des Ammoniumgehalts jedoch durch Vergleich des Ergebnisses der Messung im Absorptionsbereich des Nitroprussids mit einem vorab definierten Schwellenwert. Liegt das Messergebnis oberhalb dieses Schwellenwerts, ist das Ergebnis der Bestimmung des Ammoniumgehalts der Probe plausibel. Liegt das Ergebnis der Messung im Absorptionsbereich des Nitroprussids unterhalb des Schwellenwerts, muss die Plausibilität des Ergebnisses der Bestimmung des Ammoniumgehalts der Probe angezweifelt werden und entsprechend z.B. durch Messung von Verdünnungsreihen überprüft werden.

Der Schwellenwert kann vom Fachmann derart festgelegt werden, dass er sicherstellt, dass noch ausreichend Nitroprussid in der zu vermessenden Mischung vorhanden ist. Typischerweise sind 40%, bevorzugt ca. 50% der ursprünglich zugegebenen Menge an Nitroprussid ausreichend.

Bevorzugt wird der Schwellenwert vorab festgelegt durch Bereitstellen einer Blindwert-Probe, die kein Ammonium enthält, Einstellen auf alkalischen pH-Wert und Versetzen dieser Blindwert-Probe mit einem Chlorierungsmittel, einem Phenolderivat und Nitroprussid, so dass sie bezüglich pH-Wert, Chlorierungsmittel, Phenolderivat und Nitroprussid dieselbe Zusammensetzung und Konzentration aufweist wie die mit diesen Reagenzien versetzte wässrige Probe, deren Ammoniumgehalt bestimmt werden soll. Dann wird die Extinktion der Blindwert-Probe im Absorptionsbereich des Nitroprussids gemessen. Der Schwellenwert wird dann durch Halbieren des Extinktionswerts berechnet. Bevorzugt erfolgt Bestimmungen der Extinktion des Nitroprussids bei der wässrigen Probe und bei der Blindwert-Probe bei derselben Wellenlänge.

Die Durchführung von photometrischen Messungen ist dem Fachmann bekannt. Die photometrische Messung erfolgt typischerweise durch Messung der Extinktion bei zumindest zwei Wellenlängen im Bereich von 300 - 700 nm. Geeignet hierfür sind alle Photometer, die für Messungen zwischen 300 nm und 700 nm geeignet sind.

In der Regel wird die wässrige Probe für die Messung der Extinktion in eine Küvette gegeben.

Eine Küvette ist erfindungsgemäß ein Gefäß, in dem photometrische Messungen durchgeführt werden können. Typischerweise bestehen Küvetten aus Quarz, Glas oder Kunststoff und haben mindestens zwei planparallele Seitenflächen oder sind rund. Durch die Wahl der Küvette bzw. der Dicke der Küvette kann die Extinktion bei gleicher Zusammensetzung der zu vermessenden Probe verringert bzw. erhöht werden.

Der wässrigen Probe können vor oder nach dem alkalisch machen der Probe neben den Reagenzien zur Bestimmung des Ammonium-Gehaltes weitere Reagenzien zugesetzt werden. Das kann beispielsweise notwendig sein, um in der Probe vorhandene, andere Bestandteile daran zu hindern, bei den für die Bestimmung notwendigen hohen pH Werten auszufallen, oder um eine Störung der Bildung des Indophenols zu verhindern. Beispiele für derartige Reagenzien sind EDTA oder Trinatriumcitrat.

Die vorliegende Erfindung ermöglicht eine große Reduktion des Arbeitsaufwands bei der Bestimmung des Ammoniumgehalts nach Berthelot. Insbesondere in Laboren, in denen täglich viele Proben vermessen werden, kann der Arbeitsaufwand stark reduziert werden. Verdünnungsreihen müssen nur noch dann zur finalen Klärung durchgeführt werden, wenn die Plausibilitätsprüfung nach dem erfindungsgemäßen Verfahren zeigt, dass die Plausibilität angezweifelt werden muss.

Die vorliegende Beschreibung ermöglicht es dem Fachmann die Erfindung umfassend anzuwenden und auszuführen. Auch ohne weitere Ausführungen wird daher davon ausgegangen, dass ein Fachmann die obige Beschreibung im weitesten Umfang nutzen kann.

Bei etwaigen Unklarheiten versteht es sich von selbst, die zitierten Veröffentlichungen und Patentliteratur heranzuziehen.

Zum besseren Verständnis und zur Verdeutlichung der Erfindung werden im Folgenden Beispiele gegeben. Diese Beispiele dienen auch zur Veranschaulichung möglicher Varianten. Aufgrund der allgemeinen Gültigkeit des beschriebenen Erfindungsprinzips sind die Beispiele jedoch nicht geeignet, den Schutzbereich der vorliegenden Anmeldung nur auf diese zu reduzieren.

Weiterhin versteht es sich für den Fachmann von selbst, dass sich sowohl in den gegebenen Beispielen als auch in der übrigen Beschreibung die in den Zusammensetzungen enthaltenen Komponentenmengen in der Summe immer nur zu 100 Gew. bzw. mol-% bezogen auf die Gesamtzusammensetzung aufaddieren und nicht darüber hinausgehen können, auch wenn sich aus den angegebenen Prozentbereichen höhere Werte ergeben könnten. Sofern nichts anderes angegeben ist, gelten %-Angaben als Gew., mit Ausnahme von Verhältnissen, die in Volumenangaben wiedergegeben sind, wie beispielsweise Elutionsmittel, zu deren Herstellung Lösungsmittel in bestimmten Volumenverhältnissen im Gemisch verwendet werden.

Die in den Beispielen und der Beschreibung sowie in den Ansprüchen gegebenen Temperaturen gelten immer in °C.

### Beispiele

### Beispiel nach dem Stand der Technik

Die Bestimmung des Ammoniumgehaltes von Proben mit unterschiedlichem Gehalt an Ammonium wird entsprechend eines gebrauchsfertigen Testsatzes zur Bestimmung von Ammonium der Firma Merck durchgeführt. Hierbei wird Artikelnummer 1147390001 Spectroquant^{®} Ammonium-Küvettentest verwendet. Das Phenolderivat ist in diesem Fall 2-Chlorphenol. Die Messung findet bei einer Wellenlänge von 703 nm statt. Das Ergebnis ist in Abbildung 1 dargestellt.

Es ist zu erkennen, dass bis zum einem Ammoniumgehalt von ca. 20 bis 30 mg/l die Extinktion steigt. Danach nimmt sie trotz des höheren Ammoniumgehalts wieder ab. Die beiden Messungen bei einem Ammoniumgehalt über 100 mg/l ergeben wesentlich zu niedrige Extinktionswerte.

### Erfindungsgemäßes Beispiel:

**Tabelle 1**

| NH₄-N [mg/l] | A₇₀₃ₙₘ (Indophenol blau) | A₃₉₇ₙₘ (Nitroprussid) |
|---|---|---|
| 0 | 0,057 | 3,854 |
| 0,2 | 0,279 | 3,921 |
| 2 | 2,281 | 4,227 |
| 20 | 4,487 | 3,733 |
| 200 | 1,828 | 1,124 |
| 2000 | 0,039 | 0,699 |
| Schwellenwert 1: A₇₀₃ₙₘ ≤ 2,400 | | |
| Schwellenwert 2: A₃₉₇ₙₘ ≥ 3,400 | | |

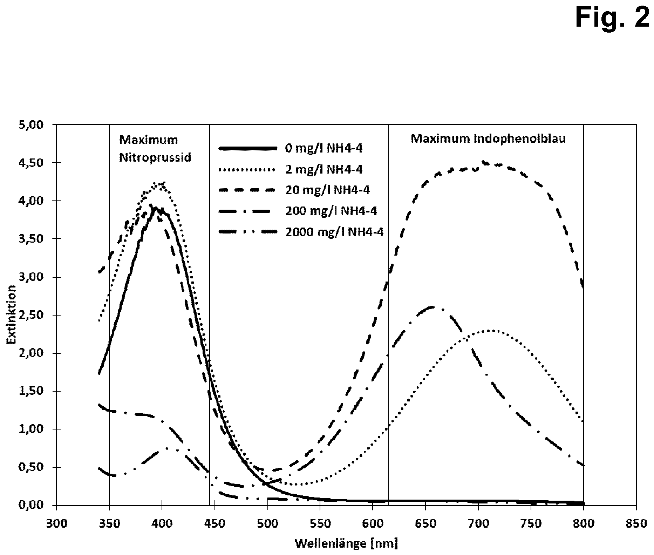

Die Werte der Tabelle 1 bzw. die in Abbildung 2 dargestellten Kurven wurden mithilfe eines gebrauchsfertigen Testsatzes zur Bestimmung von Ammonium der Firma Merck ermittelt. Hierbei wurde Artikelnummer 1147390001 Spectroquant^{®} Ammonium-Küvettentest verwendet. Der Testsatz ist für Proben innerhalb des Messbereichs 0,010 - 2,000 mg/l NH₄-N geeignet. Analysiert wurden Standardproben mit einem Gehalt von 0 / 0,20 / 2,00 / 20,0 / 200 und 2000 mg/l NH₄-N. Als Standardsubstanz wurde Ammoniumchlorid NH₄Cl eingesetzt. Um eine Lösung von 2000 mg/l NH4-N herzustellen, müssen 7,638 g NH₄Cl in 1000 ml dest. Wasser gelöst werden. Die anderen aufgeführten Konzentrationen werden durch Verdünnung mit dest. Wasser aus dieser Lösung hergestellt. Der Testsatz zeichnet sich dadurch aus, dass die benötigten Reagenzien gebrauchsfertig vorliegen. Die Durchführung erfolgt in sogenannten Rundküvetten mit einem Durchmesser von 16 mm. In der Rundküvette sind bereits 1,5 ml eines Reagenz vorgelegt. Dieses Reagenz enthält die Natronlauge, sowie 2-Chlorphenol als Phenolderivat. Beide Komponenten sind in dest. Wasser gelöst.

Zum vorgelegten Reagenz in der Rundküvette werden im ersten Reaktionsschritt 5,0 ml Probe bzw. Standardlösung gegeben und gemischt. Im zweiten Reaktionsschritt wird mit einem Dosierlöffel ein Feststoffreagenz zugegeben und unter Schütteln gelöst und homogen gemischt. Das Feststoffreagenz enthält die weiteren erforderlichen Reaktionskomponenten Nitroprussid und Dichlorisocyanursäure sowie Füllstoffe.

Nachdem alle Komponenten miteinander homogen gemischt sind, beginnt eine Reaktionszeit von 15 Minuten.

Nach Ablauf der 15 Minuten wird die Extinktion der Rundküvette an einem Spektralphotometer bei 703 nm und 397 nm gemessen. Die Messung erfolgt gegen eine Referenzküvette des gleichen Typs, die mit dest. Wasser gefüllt ist.

Die Messung bei 703 nm zeigt die Extinktion des entstandenen Indophenols bei den vorliegenden Reaktionsbedingungen (pH-Wert und Reaktionszeit), die Messung bei 397 nm zeigt die Extinktion des Nitroprussid nach Ablauf der Reaktion bei den vorliegenden

Reaktionsbedingungen (pH-Wert und Reaktionszeit).

Man sieht, dass die Extinktion des Indophenols in dem angegebenen Messbereich von 0,010 - 2,000 mg/l NH₄-N proportional ansteigt. Auch bei einer Konzentration von 20 mg/l NH₄-N erfolgt ein weiterer Anstieg der Extinktion des Indophenols. Dieser Anstieg ist nicht proportional zur Konzentration des NH₄-N. Bei 200 mg/l NH₄-N liegt die Extinktion bei 703 nm unter der Extinktion des Standards mit einem Gehalt von 2 mg/l NH₄-N. Dies würde im Extremfalle zu den geschilderten Fehlinterpretationen führen. Bei der gleichzeitig durchgeführten Messung bei 397 nm zur Detektion des vorhandenen Nitroprussids kann man sehen, dass bei sehr hohen Konzentrationen die Extinktion stark abnimmt. Über eine verknüpfte Interpretation der Messungen beider Wellenlängen, lässt sich nun eine Plausibilitätsregel aufstellen. Diese könnte für das gezeigt Beispiel wie folgt lauten:
Messung plausibel wenn Extinktion bei 703 nm < 2,400 A und Extinktion bei 397 nm >3,400 A ist.

## Patentansprüche

1. Verfahren zur Bestimmung des Ammonium-Gehaltes von wässrigen Proben, das neben dem Messergebnis einer einzelnen Messung direkt auch Informationen zur Plausibilität dieses Messergebnisses liefert, wobei
a) die Probe bei alkalischem pH-Wert mit einem Chlorierungsmittel, einem Phenolderivat und Nitroprussid versetzt wird,
b) die Extinktion der in Schritt a) erhaltenen Mischung im Absorptionsbereich des entstehenden blauen Farbstoffs und im Absorptionsbereich des Nitroprussids bestimmt wird, wobei aus der Extinktion im Absorptionsbereich des entstehenden blauen Farbstoffs der Ammonium-Gehalt bestimmt wird und aus der Extinktion im Absorptionsbereichs des Nitroprussids die Plausibilität ermittelt wird.

2. Verfahren nach Anspruch 1, **dadurch gekennzeichnet, dass** das Chlorierungsmittel Dichlorisocyanursäure ist.

3. Verfahren nach einem der Ansprüche 1 oder 2, **dadurch gekennzeichnet, dass** das Phenolderivat 2-Chlorphenol ist.

4. Verfahren nach einem oder mehreren der Ansprüche 1 bis 3, **dadurch gekennzeichnet, dass** der alkalische pH in Schritt a) erzeugt wird durch Zugabe von Natronlauge.

5. Verfahren nach einem oder mehreren der Ansprüche 1 bis 4, **dadurch gekennzeichnet, dass** der pH-Wert in Schritt a) zwischen pH 11 und pH 12 eingestellt wird.

6. Verfahren nach einem oder mehreren der Ansprüche 1 bis 5, **dadurch gekennzeichnet, dass** die Bestimmung der Extinktion in Schritt b) mit einem Spektralphotometer erfolgt.

7. Verfahren nach einem oder mehreren der Ansprüche 1 bis 6, **dadurch gekennzeichnet, dass** die Bestimmung der Extinktion derart erfolgen, dass eine photometrische Messung in dem Wellenlängenbereich zwischen 350 und 450 nm vorgenommen wird und eine photometrische Messung in dem Wellenlängenbereich zwischen 600 und 800 nm.

8. Verfahren zur Bestimmung der Plausibilität des Messergebnisses einer Bestimmung des Ammonium-Gehaltes von wässrigen Proben mittels Durchführung eines Verfahrens nach Anspruch 1, wobei die Extinktion der Probe im Absorptionsbereich des entstehenden blauen Farbstoffs nur dann als plausibles Messergebnis angesehen wird, wenn die Extinktion im Absorptionsbereich des Nitroprussids oberhalb eines vorab zu bestimmenden Schwellenwert liegt, der das Vorhandensein von mindestens 50% der in Schritt a) zugegebenen Menge an Nitroprussid anzeigt.

9. Verfahren nach Anspruch 8, **dadurch gekennzeichnet, dass** der Schwellenwert bestimmt wird durch
i. Bereitstellen einer Blindwert-Probe, in Form einer wässrigen Lösung, die kein Ammonium enthält, mit alkalischen pH-Wert und versetzen dieser wässrigen Lösung mit einem Chlorierungsmittel, einem Phenolderivat und Nitroprussid, so dass sie bezüglich pH-Wert, Chlorierungsmittel, Phenolderivat und Nitroprussid dieselbe Zusammensetzung und Konzentration aufweist wie die Mischung in Schritt b), deren Extinktion bestimmt wird
ii. Bestimmung der Extinktion der Blindwert-Probe im Absorptionsbereich des Nitroprussids
iii. Berechnung des Schwellenwertes durch Halbieren des in Schritt ii.) bestimmten Extinktionswerts.

10. Verfahren nach Anspruch 9, **dadurch gekennzeichnet, dass** das Chlorierungsmittel Dichlorisocyanursäure ist.

11. Verfahren nach einem der Ansprüche 9 oder 10, **dadurch gekennzeichnet, dass** das Phenolderivat 2-Chlorphenol ist.

12. Verfahren nach einem oder mehreren der Ansprüche 9 bis 11, **dadurch gekennzeichnet, dass** der alkalische pH in Schritt a) erzeugt wird durch Zugabe von Natronlauge.

13. Verfahren nach einem oder mehreren der Ansprüche 9 bis 12, **dadurch gekennzeichnet, dass** der pH-Wert in Schritt a) zwischen pH 11 und pH 12 eingestellt wird.

14. Verfahren nach einem oder mehreren der Ansprüche 9 bis 13, **dadurch gekennzeichnet, dass** die Bestimmung der Extinktion des Nitroprussids in Schritt b) und Schritt ii) jeweils im Absorptionsmaximum des Nitroprussids erfolgen.

## Claims

1. Method for the determination of the ammonium content of aqueous samples which, besides the measurement result of a single measurement, also supplies directly information on the plausibility of this measurement result, where
a) a chlorinating agent, a phenol derivative and nitroprusside are added to the sample at an alkaline pH,
b) the extinction of the mixture obtained in step a) is determined in the absorption region of the blue dye formed and in the absorption region of nitroprusside, where the ammonium content is determined from the extinction in the absorption region of the blue dye formed and the plausibility is determined from the extinction in the absorption region of nitroprusside.

2. Method according to Claim 1, **characterised in that** the chlorinating agent is dichloroisocyanuric acid.

3. Method according to one of Claims 1 or 2, **characterised in that** the phenol derivative is 2-chlorophenol.

4. Method according to one or more of Claims 1 to 3, **characterised in that** the alkaline pH in step a) is generated by addition of sodium hydroxide solution.

5. Method according to one or more of Claims 1 to 4, **characterised in that** the pH in step a) is set between pH 11 and pH 12.

6. Method according to one or more of Claims 1 to 5, **characterised in that** the determination of the extinction in step b) is carried out using a spectrophotometer.

7. Method according to one or more of Claims 1 to 6, **characterised in that** the determination of the extinction is carried out by carrying out a photometric measurement in the wavelength region between 350 and 450 nm and a photometric measurement in the wavelength region between 600 and 800 nm.

8. Method for the determination of the plausibility of the measurement result of a determination of the ammonium content of aqueous samples by carrying out a method according to Claim 1, where the extinction of the sample in the absorption region of the blue dye formed is only regarded as a plausible measurement result if the extinction in the absorption region of nitroprusside is above a threshold value to be determined in advance which indicates the presence of at least 50% of the amount of nitroprusside added in step a).

9. Method according to Claim 8, **characterised in that** the threshold value is determined by
i. provision of a blank value sample, in the form of an aqueous solution which contains no ammonium, having an alkaline pH, and addition of a chlorinating agent, a phenol derivative and nitroprusside to this aqueous solution so that it has the same composition and concentration with respect to pH, chlorinating agent, phenol derivative and nitroprusside as the mixture in step b) whose extinction is being determined
ii. determination of the extinction of the blank value sample in the absorption region of nitroprusside
iii. calculation of the threshold value by halving the extinction value determined in step ii.).

10. Method according to Claim 9, **characterised in that** the chlorinating agent is dichloroisocyanuric acid.

11. Method according to one of Claims 9 or 10, **characterised in that** the phenol derivative is 2-chlorophenol.

12. Method according to one or more of Claims 9 to 11, **characterised in that** the alkaline pH in step a) is generated by addition of sodium hydroxide solution.

13. Method according to one or more of Claims 9 to 12, **characterised in that** the pH in step a) is set between pH 11 and pH 12.

14. Method according to one or more of Claims 9 to 13, **characterised in that** the determination of the extinction of nitroprusside in step b) and step ii) is in each case carried out at the absorption maximum of nitroprusside

## Revendications

1. Méthode de détermination de la teneur en ammonium d'échantillons aqueux qui, outre le résultat de mesure d'une mesure unique, fournit également directement des informations sur le caractère plausible de ce résultat de mesure, dans laquelle
a) un agent de chloration, un dérivé de phénol et du nitroprussiate sont ajoutés à l'échantillon à un pH alcalin,
b) l'extinction du mélange obtenu dans l'étape a) est déterminée dans la région d'absorption du colorant bleu formé et dans la région d'absorption du nitroprussiate, où la teneur en ammonium est déterminée à partir de l'extinction dans la région d'absorption du colorant bleu formé et le caractère plausible est déterminé à partir de l'extinction dans la région d'absorption du nitroprussiate.

2. Méthode selon la revendication 1, **caractérisée en ce que** l'agent de chloration est l'acide dichloroisocyanurique.

3. Méthode selon l'une parmi les revendications 1 ou 2, **caractérisée en ce que** le dérivé de phénol est le 2-chlorophénol.

4. Méthode selon l'une ou plusieurs parmi les revendications 1 à 3, **caractérisée en ce que** le pH alcalin dans l'étape a) est généré par l'addition d'une solution d'hydroxyde de sodium.

5. Méthode selon l'une ou plusieurs parmi les revendications 1 à 4, **caractérisée en ce que** le pH dans l'étape a) est ajusté entre pH 11 et pH 12.

6. Méthode selon l'une ou plusieurs parmi les revendications 1 à 5, **caractérisée en ce que** la détermination de l'extinction dans l'étape b) est effectuée à l'aide d'un spectrophotomètre.

7. Méthode selon l'une ou plusieurs parmi les revendications 1 à 6, **caractérisée en ce que** la détermination de l'extinction est effectuée par la mise en oeuvre d'une mesure photométrique dans la région de longueur d'onde comprise entre 350 et 450 nm et d'une mesure photométrique dans la région de longueur d'onde comprise entre 600 et 800 nm.

8. Méthode de détermination du caractère plausible du résultat de mesure d'une détermination de la teneur en ammonium d'échantillons aqueux par la mise en oeuvre d'une méthode selon la revendication 1, où l'extinction de l'échantillon dans la région d'absorption du colorant bleu formé n'est seulement considérée comme résultat plausible de mesure si l'extinction dans la région d'absorption du nitroprussiate est supérieure à une valeur seuil, à déterminer à l'avance, qui indique la présence d'au moins 50% de la quantité de nitroprussiate ajoutée dans l'étape a).

9. Méthode selon la revendication 8, **caractérisée en ce que** la valeur seuil est déterminée par
i. la mise à disposition d'un échantillon à valeur de blanc, sous la forme d'une solution aqueuse qui ne contient pas d'ammonium, présentant un pH alcalin, et l'addition d'un agent de chloration, d'un dérivé de phénol et de nitroprussiate à cette solution aqueuse de façon à ce qu'elle présente les mêmes composition et concentration en ce qui concerne le pH, l'agent de chloration, le dérivé de phénol et le nitroprussiate, que le mélange dans l'étape b) dont l'extinction doit être déterminée ;
ii. la détermination de l'extinction de l'échantillon à valeur de blanc dans la région d'absorption du nitroprussiate ;
iii. le calcul de la valeur seuil en divisant par deux la valeur d'extinction déterminée dans l'étape ii.).

10. Méthode selon la revendication 9, **caractérisée en ce que** l'agent de chloration est l'acide dichloroisocyanurique.

11. Méthode selon l'une parmi les revendications 9 ou 10, **caractérisée en ce que** le dérivé de phénol est le 2-chlorophénol.

12. Méthode selon l'une ou plusieurs parmi les revendications 9 à 11, **caractérisée en ce que** le pH alcalin dans l'étape a) est généré par l'addition d'une solution d'hydroxyde de sodium.

13. Méthode selon l'une ou plusieurs parmi les revendications 9 à 12, **caractérisée en ce que** le pH dans l'étape a) est ajusté entre pH 11 et pH 12.

14. Méthode selon l'une ou plusieurs parmi les revendications 9 à 13, **caractérisée en ce que** la détermination de l'extinction du nitroprussiate dans l'étape b) et l'étape ii) est effectuée dans chaque cas au niveau de l'absorption maximale du nitroprussiate.
